# EUROPEAN PATENT APPLICATION

(11) **EP 4 548 923 A1**
(43) Date of publication of application: **07.05.2025**
(21) Application number: 23795696.6
(22) Date of filing: 25.04.2023
(51) Int. Cl.: A61K 31/715, A61K 31/717, A61K 31/737

(54) **COMPOSITION FOR INTESTINAL REHABILITATION**

(30) Priority: 26.04.2022 ES 202230379
(71) Applicant: Servicio Andaluz de Salud, 41071 Sevilla (ES); Universidad de Granada, 18071 Granada (ES)
(72) Inventor: CARRILLO ACOSTA, Alberto, 41071 Sevilla (ES); GÓMEZ LLORENTE, Carolina, 18071 Granada Granada (ES)
(74) Representative: San Martin Alarcia, Esther
(86) International application number: PCT/ES2023/070261
(87) International publication number: WO 2023/209258

(57) **Abstract**

The present invention relates to a composition for re-establishing the formation of functional faeces and restoring the bowel function in patients with temporary derivative ileostomy, said composition based on soluble fibres and comprising at least one prebiotic oligosaccharide and a soluble cellulose derivative.

## Description

### FIELD OF THE INVENTION

The present invention is within the field of Medicine and in particular Surgery, and concerns the application of a new composition for the restoration of functional faeces formation and the restoration of defecatory function in patients with temporary bypass ileostomy.

### STATE OF THE ART

Currently, the incidence of rectal cancer in the European Union is 150,000 cases per year, or 15-25 cases per 100,000 inhabitants per year, and is expected to increase in the near future. With advances in surgical technique, it is becoming increasingly feasible to perform sphincter-preserving surgery rather than amputation, with more and more extreme anastomoses being performed. This fact explains why the prevalence of functional alterations in defecation after surgery has increased proportionally.

In this context, defecatory dysfunction may be due to changes resulting from treatment (surgery, chemo-radiotherapy), scarring phenomena and local complications (dehiscence, abscess, etc.), the time elapsed since reconstruction, the absence of early rehabilitation (a preventive actor of sequelae) and the absence of faecal transit at the colorectal level.

Rectal surgery modifies the anorectal architecture, alters the nervous system of the pelvis and replaces an organ dedicated to defecation with one that is not. In addition, in 77% of patients who undergo rectal cancer surgery, a protective ileostomy is required to prevent the passage of faeces to the rest of the colon [1].

Loop bypass ileostomy is a surgical resource to protect an intestinal anastomosis, performed distally, when the context is suboptimal and the possibility of dehiscence is greater than acceptable. It consists of removing the terminal ileus, opening it and suturing its two mouths to the skin to interrupt the passage of faeces through the colon, rectum and anus. The aim is to avoid complications arising from the passage of faeces through a risk area.

Once the postoperative period necessary for the healing of the operation has passed, usually 3 - 4 weeks if there have been no complications, the patient is a candidate for intestinal transit reconstruction. However, this procedure is usually delayed until after 6 months due to the need for adjuvant treatment. Even so, 18 months after surgery, stoma reconstruction has not been performed in 28% of electively operated patients, and in up to 59% of urgent cases **[1].**

The absence of intestinal faecal transit at the colorectal level produces a series of negative consequences if maintained over time, among them:
- . Dysbiosis; the absence of faeces alters the intestinal microbiota **[2],** defined as the set of microorganisms that inhabit the intestine, causing an increase in Proteobacteria. Dysbiosis has been associated with increased postoperative morbidity **[3],** *Clostridium Difficile* infections **[4],** paralytic ileus and altered bowel habit.
- . Mucosal atrophy; the lack of intestinal contents in contact with the mucosa prevents nutrition of the colonocytes, which depends on short-chain fatty acids (butyrate, propionate and acetate) produced by the bacterial flora when fermenting the undigested fibre in previous sections. The absence of substrate to maintain the metabolism of the microbiota leads to atrophy and inflammation of the gut mucosa, known as exclusion colitis **[5].**
- . Neuromuscular atrophy; by abolishing intestinal faecal transit, the normal functioning of the rest of the colon, rectum and pelvic floor is prevented, causing involution of the colorectal and pelvic musculature due to decreased motor activity **[6].**

Finally, as the last intestinal tract does not receive faeces, it is not possible to assess whether the defecatory function, with the new configuration, will be adequate or not.

Thus, when intestinal transit is finally reconstructed, the resulting defecatory function may be severely impaired in up to 47% of patients.

Under normal conditions, 1,500 ml of intestinal contents (chyme) reach the cecum daily. After the process of absorption and fermentation of the intestinal contents, between 80 and 300 grams of faeces are produced daily. The composition of faeces is mostly watery, the dry weight being about 25%. The dry weight consists mainly of bacterial mass, undigested dietary products and other inorganic remains (calcium, potassium, etc.).

To avoid the consequences of the above-mentioned absence of colorectal and pelvic faecal transit, the use of external prostheses to re-establish transit continuity and/or the manual instillation (dropwise administration) of the intestinal chyme itself has been proposed **[7, 8, 9].**

Anterograde instillation of saline, saline with a thickening agent **[27],** isomolar solution of saline and sucrose, liquid diet solution (vitamins, minerals, proteins and fats) and short-chain fatty acids **[3, 10]** have been described with the intention of reducing postoperative morbidity.

The use of anhydrous sodium dihydrogen phosphate (Casen enema) through the anus has also been described.

**All of these substances have been used with the aim of stimulating the bowel to decrease the rate of post-surgical ileus, characterized by a delay in the return of coordinated bowel motility after surgery.**

Aminosalicylate (5-ASA) and corticosteroid enemas have been tried to reduce the rate of exclusion colitis with varying effectiveness **[11, 12, 13].**

Instillation of dietary solutions to reduce mucosal inflammation and prepare the colon for recovery of faecal transit after colostomy closure has been described in patients with temporary colostomy. The compositions used contained both soluble and insoluble fibres, as well as proteins, lipids and minerals, with the fibre percentage of the composition being very low at 5% **[14].**

Early enteral nutrition has also been tested in patients undergoing major colorectal surgery, in which hyperproteic and hypercaloric nutritional compositions with soluble and insoluble fibres are administered through a nasojejunal cannula into the intestine as a step prior to administration of a normal diet **[25].**

Dietary fibre is known to affect gastric emptying, digestion transit time or faecal bulk. Mixtures of fermentable oligosaccharides with non-fermentable fibre have been used in small proportions as dietary supplements in dog foods **[24]** and combinations of soluble and insoluble fibres have also been used as an anti-inflammatory dietary supplement in humans **[26]** to improve nutrient metabolism, intestinal functions and intestinal microecology, to try to restore intestinal motility and to prevent constipation. However, the effectiveness of these compounds has been studied in healthy individuals, in the context of improving intestinal function that is not pathological, let alone disrupted by an ileostomy.

Finally, a review article has proposed the application of saline or polyethylene glycol (PEG) enemas through the efferent orifice of the ileostomy in an attempt to reduce the incidence of Low Anterior Resection Syndrome (LARS), although it does not conduct studies in this direction **[15].**

Low Anterior Resection Syndrome or LARS is one of the potential sequelae of colorectal surgery, consisting of defecatory dysfunction and presenting as a cluster of symptoms including defecatory urgency, faecal incontinence and faeces fragmentation, with repeated, incomplete or difficult bowel movements.

The state of the art describes the management of Low Anterior Resection Syndrome after ileostomy reconstruction surgery has been performed and once the presence of the syndrome has been diagnosed, as there is currently no method to diagnose the syndrome prior to transit reconstruction. The way to predict the resulting defecatory capacity after colorectal surgery is based on the risk factors described in the literature associated with LARS. Preoperative assessment of these factors does not preclude the prevalence of severe LARS after bowel transit reconstruction to be up to 47%.

**Neither the compounds nor techniques mentioned here have been described as being aimed at restoring functional faeces formation and physiological defecatory function in patients with a bypass ileostomy.** That is, none of these compounds or techniques has resulted in the generation of functional faeces in a patient with an ileostomy-derived bowel transit interruption.

In addition, it should be noted that a substance instilled through the ileostomy may be absorbed by the colonic mucosa, transformed by the microbiota in the intestinal lumen or expelled unchanged.

The substances previously studied and mentioned here have been used with the intention of being absorbed by the colonic mucosa (anti-inflammatory, nutritional, etc.) or to trigger pathological peristalsis leading to watery diarrhea (saline enemas). **In no case are the examples of the prior art designed to restore functional faeces formation, physiological peristalsis or physiological or normal defecatory function.**

**To date, no product, natural or synthetic, has been identified that is capable of forming functional faeces in patients with terminal ileostomy in a way that restores normal defecatory function.**

Functional faeces are defined as those that have the capacity to restore nutrition to the colorectal mucosa and to re-establish sufficient neuromuscular activity in the excluded colonic segment and pelvic musculature, similar to the way natural faeces act **[16].** For this purpose, it is considered that faeces should be similar in size to those produced prior to stoma creation, of adequate consistency (grades 2 to 4 on the Bristol scale) **[17]** and with a normal microbiota as described in the literature (mainly Bacteroidetes, Firmicutes and Proteobacteria) **[18, 19].**

### DESCRIPTION OF THE INVENTION

A **composition,** hereinafter "composition of the invention", with different types of **soluble fibre** comprising at least **one prebiotic oligosaccharide, a cellulose derivative and a humectant** has been developed which by instillation through the distal orifice of the ileostomy is able to **restore functional faeces production and physiological defecatory function in patients with temporary bypass ileostomy prior to definitive transit reconstruction.**

This implies that while the digestive transit is interrupted by the ileostomy, patients to whom the composition of the invention is applied through the distal orifice of the ileostomy are able to produce functional faeces and recover physiological defecatory function.

It should be noted that none of the compounds mentioned in the current state of the art is able to reactivate the formation of functional faeces and thus the physiological defecatory function in patients undergoing ileostomy. In these cases there is an interruption of the intestinal transit whereby the chyme is expelled out of the body through the ileostomy collection bag. To date, only compounds or treatments have been proposed to try to alleviate the negative effects resulting from the interruption of intestinal transit, the absence of functional faeces production, and thus, the lack of physiological defecatory function.

Compounds including a variety of components (soluble fibres, insoluble fibres, proteins, lipids and minerals) have been proposed for use in patients with temporary colostomy **[14]** and in patients with enteral feeding after major colorectal surgeries **[25].** However, these differ from the composition of the invention in both their composition and their proposed purpose and use. On the one hand, the proportion of fibres of which they are composed (soluble and insoluble) barely represent 5% and 2% of the total respectively, whereas fibres, in this case soluble, are the main component of the composition of the invention.

In addition, the administration of these compounds to colostomy patients aims, in one case, to decrease colonic inflammation **[14]** and, in the other case, to serve as a preliminary step to the restoration of a normal diet **[25],** but neither of these compositions is able to produce functional faeces while intestinal transit is interrupted by the colostomy.

It should be borne in mind that digestion and therefore formation of faeces as such is not possible in the colon, as its function is merely that of absorption of water, electrolytes and minerals. Therefore, a compound administered by colostomy in the proposed locations (sigmoid colostomy, transverse colostomy, etc.) cannot in any case generate functional faeces on its own. Thus, in the case of colorectal surgery involving an ileostomy, the formation of functional faeces and the re-establishment of defecatory function is not possible until intestinal transit is restored.

In the present case, where the interruption of intestinal transit is caused by an ileostomy in the small intestine, the composition of the invention, when administered by instillation through the distal orifice of the ileostomy, restores the formation of functional faeces, defined as biologically active faeces (presence of physiological bacterial flora), in adequate quantity and consistency, allowing the restoration of intestinal peristalsis, defecatory function and nutrition of the colorectal mucosa, because:
- It produces a mechanical effect similar to that of natural faeces, preventing colorectal and pelvic muscle atrophy, stimulating the pelvic nervous system and modifying post-surgical healing to a pro-functional state before they are permanently configured.
- It serves as nutrition for the maintenance of the normal intestinal microbiota (mainly lactobacillus and bifidobacteria). The normal microbiota in turn nourishes the colonocytes, as they are the natural producers of Short Chain Fatty Acids (butyrate, propionate, acetate), preventing atrophy of the colorectal mucosa (exclusion colitis). They also regulate pH, preventing colonization by pathogens such as *C*. *difficile.*

Furthermore, thanks to the inclusion of humectant compounds, and by adjusting their proportion within the composition of the invention, different rehabilitation patterns can be established by producing more or less liquid faeces (level of colorectal stress).

The composition of the invention also makes it possible to restore colorectal nutrition thanks to the maintenance of the colonic microbiota, preventing the onset of exclusion colitis.

Finally, applying the treatment for the appropriate time allows diagnosing, before reconstructing the intestinal transit, which patients have a Severe Anterior Resection Syndrome, and therefore are not ideal candidates for intestinal reconstruction.

**A first aspect of the invention** relates to a composition for use in a treatment method for the restoration of functional faeces formation and physiological defecatory function in patients with temporary bypass ileostomy comprising **various types of soluble fibres, namely at least one prebiotic oligosaccharide and one soluble cellulose derivative.**

Prebiotic oligosaccharides are soluble fibres that are not absorbed by the intestine but are metabolized by the colonic intestinal flora. They serve as nutrition for the maintenance of the normal colonic microbiota.

In a preferred composition, the prebiotic oligosaccharide is **inulin** and/or **galacto-oligosaccharide (GOS).**

In a still more preferred composition, the inulin is **oligofructose and/or polyfructose.**

In another preferred composition, the soluble cellulose derivative is **methyl cellulose, hydroxy-propyl methyl cellulose and/or carboxy-methyl cellulose.**

In a preferred embodiment of the composition of the invention, the invention further comprises a **humectant.**

Humectants are soluble fibres capable of modifying the water-carrying capacity, varying the final consistency of the faeces. In this way, different rehabilitation patterns can be established by producing more or less liquid faeces.

In a more preferred composition, the humectant is **polydextrose, mucilage, gum, starch, alginate and/or agar.**

In a still more preferred composition, the humectant is **polydextrose.**

In a still more preferred composition, the proportion of the components is as follows: **soluble cellulose derivative (50-70%) inulin (50-30%), GOS (0-5%) and humectant (0-10%).**

In another preferred embodiment of the invention, the composition further comprises **insoluble fibre.**

Insoluble fibres are neither metabolized nor reabsorbed. They produce a mechanical effect similar to that of natural faeces, preventing colorectal and pelvic muscle atrophy, stimulating the pelvic nervous system and modifying post-surgical healing to a pro-functional state.

Preferably, the insoluble fibres are insoluble cellulose and its derivatives, insoluble hemicellulose and/or lignin.

In a preferred embodiment of the invention, the composition further comprises **Medium Chain Triglycerides and/or antioxidants.**

In a still more preferred embodiment, the Medium Chain Triglycerides and/or antioxidants are in a proportion of up to 5% of the composition.

Medium-chain triglycerides (MCT) are esters of medium-chain fatty acids (between 8 and 12 carbon atoms) and glycerol (1,2,3-propanetriol); directly nourish colonocytes, improving nutritional coverage in the early stages of rehabilitation. Antioxidants reduce oxidative stress.

In a preferred embodiment of the invention, the composition further comprises other substances of nutritional interest such as inorganic products to supplement absorption by the colon, namely **electrolytes** (such as sodium, potassium, chlorine and/or magnesium), **calcium phosphate and/or ferric phosphate.**

In a still more preferred composition, these inorganic products are in a proportion of up to 2% of the composition.

In a preferred embodiment of the invention, the composition further comprises products for functional studies such as **contrast media for MRI, X-ray scanning or others.**

The composition of the invention can be applied in routine clinical practice for intestinal rehabilitation by restoring faeces formation, physiological peristalsis and defecatory function, restoration of nutrition of the colorectal mucosa, rehabilitation of the physiological colonic microbiota and treatment or prevention of exclusion colitis. Treatment is preferably applied on a daily basis until normal intestinal transit is restored.

By applying the treatment for the appropriate time, it is possible to diagnose, before reconstructing the intestinal transit, which patients have a Severe Anterior Resection Syndrome, and therefore are not ideal candidates for intestinal reconstruction.

It is determined that there is a risk of Severe Anterior Resection Syndrome in those patients who do not respond to treatment with the composition of the invention.

The evaluation of the response to treatment is carried out by assessing the defecatory function resulting after treatment with the composition of the invention for an appropriate period of time, usually six months from the ileostomy until its reconstruction is assessed. The scale used to assess defecation is the "LARS score", where through a questionnaire a score between 0 and 42 can be obtained, and patients can be classified into three categories a) No LARS (0-20); b) minor LARS (21-29); c) major LARS (30-42) with a direct affectation of the quality of life.

Improvements in the LARS score of patients have been observed after 1 month of treatment with the composition of the invention.

Patients at risk for Severe Anterior Resection Syndrome are considered to be those with major or severe LARS (>30 points) after a 6-month period of treatment with the composition of the invention.

### DESCRIPTION OF THE FIGURES

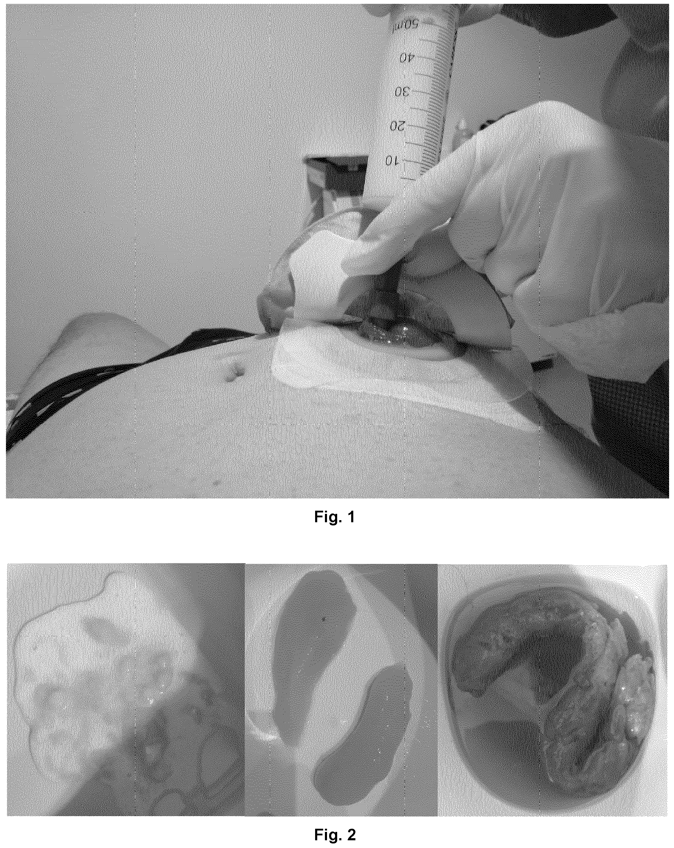

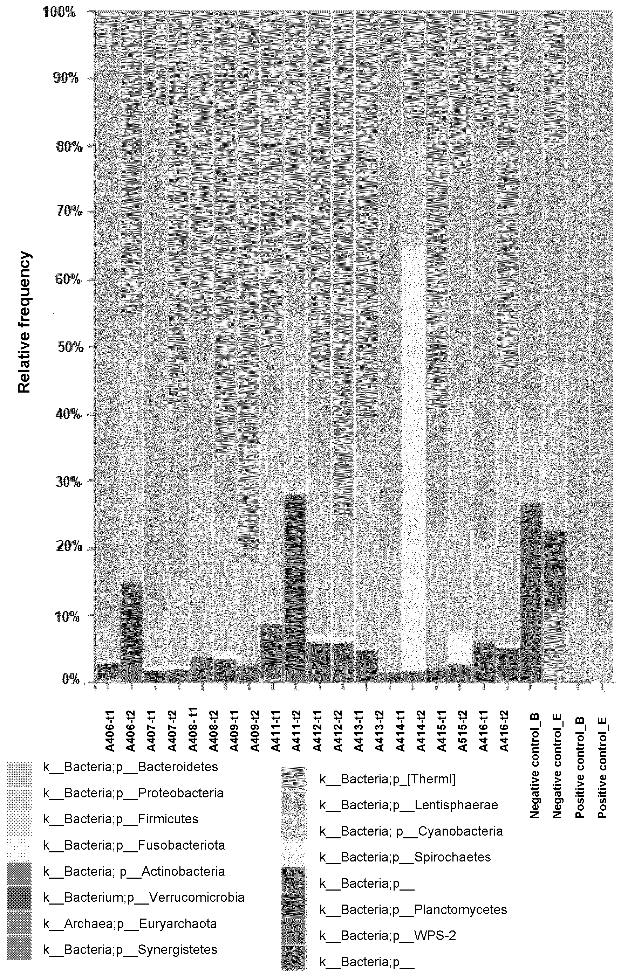

**Fig. 1****.** Instillation of the composition of the invention trans-ileostomy by catheter.
**Fig. 2****.** Experimental faeces: A) Bristol type 6. B) Bristol type 5. C) Bristol type 4.
**Fig. 3.** Composition of the gut microbiota. The figure shows the relative abundance of bacteria at the phylum level. Each column represents one participant. t1: time 1, at the start of treatment. t2: time 2, at the end of treatment after one month. Negative and positive controls correspond to sequencing controls.
**Fig. 4****.** Relative abundance of the Proteobacteria phylum throughout the study. 1: time 1, at the start of treatment. 2: time 2, at the end of treatment after one month. The solid line indicates the mean relative abundance.
**Fig. 5****.** Relative abundance of the phylum Firmicutes throughout the study. 1: time 1, at the start of treatment. 2: time 2, at the end of treatment after one month. The solid line indicates the mean relative abundance.

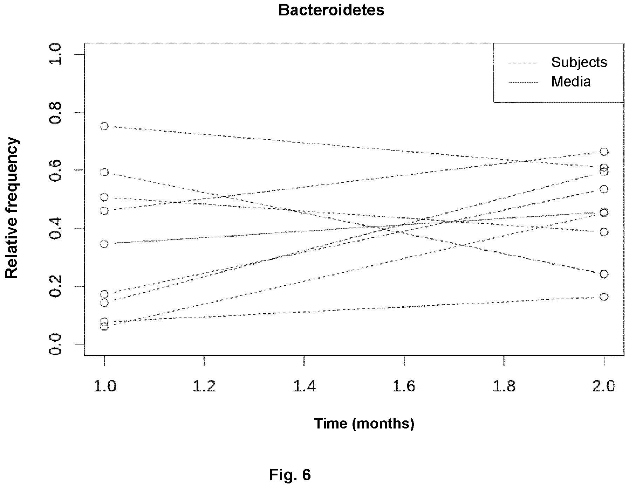

**Fig. 6****.** Relative abundance of Bacteroidetes phylum throughout the study. 1: time 1, at the start of treatment. 2: time 2, at the end of the treatment after one month. The solid line indicates the mean relative abundance.

### EXAMPLES OF THE INVENTION

In a nutritional intervention study, patients were given a product composed of soluble fibre. None of the components is associated with a harmful effect [20].

After dissolving the composition in water, instillation was performed through the distal orifice of the ileostomy using a 60cc syringe and a 28-30F flexible tube.

The frequency and volume of instillation was progressively increased until the target faeces was obtained.

The risks associated with instillation of the composition are very low and classified as mild for the most part. Symptoms are those of a large meal or indigestion, such as bloating, abdominal pain, flatulence, hard faeces or diarrhea. The study was conducted in accordance with the Helsinki Guidelines and was pre-approved by the Provincial Research Ethics Committee. The study followed international, national and regional research standards for research using samples from human subjects.

Patients included in the study were those who underwent anterior rectal resection with total or partial mesorectal excision and bypass ileostomy, who met the following inclusion and exclusion criteria:
Inclusion criteria: Patients of both genders, adults over 18 years of age, who understood the study information, signed specific informed consents, and agreed to participate in the study. Patients undergoing urgent or elective rectosigmoid resection with partial or total mesorectal excision, primary anastomosis and loop ileostomy. Patients in good general health, defined as 0-1 on the ECOG functional status scale **[21].**

Exclusion criteria: pT4 tumours of the TNM classification of rectal and colon cancer **[22].** Patients who required urgent reoperation for anastomotic dehiscence, including the use of endoscopic endoluminal vacuum therapy. Patients with active synchronous oncological pathology. Patients diagnosed with inflammatory bowel disease. Cases with previous diagnosis of defecatory dysfunction (faecal incontinence or obstructive defecation). History of previous pelvic surgery. Inability or refusal to comply with study procedures or to sign the informed consent form. Recent history or presence of severe, progressive and uncontrolled hepatic, haematological, gastrointestinal, endocrine, pulmonary, cardiac, neurological, psychiatric or skin disease. Patients with renal insufficiency defined as a creatinine clearance of less than 60ml/min according to the MDRD-4 formula or a serum creatinine equal to or 1.5 above the limit of normal. Need for chronic systemic antibiotic therapy. Congenital or acquired immunodeficiency. Allergy to any of the study treatment components.

All participants were introduced to the project, offered the patient and Andalusian Public Health System Biobank (BBSSPA) information sheet and offered to participate in the project. Once their participation was accepted, their written informed consent to the project and BBSSPA was obtained.

### Instillation protocol

Composition of the invention: 20 - 40 grams. Water: 300 - 750 millilitres. Dosage: One instillation every 8 - 48 hours.

Specifically, a composition with a 60:31:9 ratio for methyl cellulose (soluble cellulose derivative), inulin (prebiotic oligosaccharide) and polydextrose (humectant) was administered.

The minimum dose necessary to obtain the target faeces was repeated for one month until the end of the study. General schedule: A schedule of 10 grams every 24 hours was started until 30 grams per day was reached in the first week. If this was not sufficient to obtain the target faeces, the following week the number of administrations was increased to every 12 or every 8 hours, until amounts of 40 - 60 grams per day or 60 - 90 grams per day, respectively, were obtained. Those patients who obtained the target faeces with only one instillation per day were able to extend the pattern to every 48 hours per week. The general pattern could be modified according to individual patient characteristics during the course of the study, e.g. poorer tolerance to instillation volumes greater than 300cc requiring more instillations of smaller amounts.

The ability of the composition of the invention to produce faeces of sufficient consistency and quantity to restore a normal defecation frequency, defined as faeces occurring between every 8 and 48 hours, with a consistency of 2 - 4 on the Bristol scale **[16, 17];** and the prebiotic and anti-inflammatory effect of the composition on the evolution of the colonic microbiota composition in patients with excluded intestinal transit were evaluated. Secondarily, the frequency and volume of instillation required to obtain the target faeces; and the difficulties and complications associated with administration of the composition were identified.

The variables analyzed during the study were: LARS scale Weekly. Defecation diary (frequency and dosage of product application, faeces frequency, faeces consistency measured according to the Bristol scale, size and colour, subjectively assessed, in comparison with their natural faeces prior to surgery). Faecal microbiota and concentration of fermentation by-products (used as an indicator of inflammatory bowel status).

Faeces samples were collected at the patient's home in sterile jars provided by the investigator and were frozen at -20°C for a maximum of 72 hours before delivery to the clinic.

The samples were then handed over to the biobank staff at the hospital. The samples were kept frozen at -80°C until analysis. Faeces samples were taken at the beginning of the study with the first faeces deposition after starting the application of the composition of the invention, and at the end of the study with the resulting faeces deposition after the last application.

The Biobank of the Andalusian Public Health System (BBSSPA) carried out the management and custody of the samples. Once the project was completed, the samples were stored within the framework of the BBSSPA.

### Determination of the composition of the gut microbiota

Samples were kept frozen at -80°C. DNA was then obtained using the QIAamp DNA faeces mimi kit (Qiagen, Barcelona, Spain). The quality and quantity of the DNA was checked spectrophotometrically (NanoDrop ND-100). 600bp corresponding to the V3-V1 variable region of the 16S rRNA gene were amplified using specific oligonucleotides (barcoding). PCR was then carried out in a final volume of 15 µl containing the barcode oligonucleotides and the specific oligonucleotides of the different bacterial genera, families and species under study. The PCR product was pyrosequenced on the Roche/454 GS titanium technology platform (Branford, CT, USA). For the analysis of the samples, the corresponding ASVs (Amplicon Sequence Variance) were obtained and normalized by rarefaction. The Greengenes database was used for taxonomic determination.

Data analysis was by protocol (APP). A descriptive analysis of the data was performed, calculating measures of central tendency and dispersion for numerical variables (mean+/- standard deviation or median and quartiles, according to normality), and for qualitative variables, absolute and relative frequencies. The data were analyzed using IBM SPSS Statistics 19 software.

The number of participants included was 10 patients. At the end of the study one of the patients was excluded for not having completed the "Patient's notebook" and the defecation diary despite having completed the complete follow-up and treatment.

### Defecatory function

After instillation of the composition of the invention 100% of the patients restored the defecatory function of biologically active faeces at the end of the study. In the first two weeks the faeces were less consistent: 55.5% Bristol type 7, 22.2% Bristol type 6, 11.1% Bristol type 4 and 11.1% Bristol type 2. Faeces obtained at the completion of the intervention period corresponded to 66.6% Bristol type 4, 22.2% Bristol type 5, and 11.1% Bristol type 2. In the first two weeks 77.7% of the faeces were considered to be of diarrheal consistency (Bristol 6-7). At the end of the study 77.7% of the faeces were considered normal (Bristol 2-4).

In terms of frequency of defecation, 11.1% defecated every 8 hours; 22.2% every 12 hours; 33.3% every 24 hours; 22.2% every 48 hours; and 11.1% every 72 hours.

88.8% of patients achieved a defecatory frequency considered normal (8-48 hours).

66.6% of patients achieved the objective of restoring optimal defecatory function in terms of frequency and consistency, established a priori. The most frequently used dosage was full application of the composition every 48 hours.

### LARS Score -Low Anterior Resection Syndrome Score

In the first week of adaptation (low initial amount), 11.1% of patients had higher LARS. The following week, with the increase in instillation volume, 22.2% of patients had lower LARS. In the third week, applying the maximum instillation volume, 22.2% had lower LARS and 22.2% had higher LARS. In the last week, after volume adjustments and stabilization, 11.1% had lower LARS and 11.1% had higher LARS.

### Microbiota analysis

Of the 10 patients included in the study, viable samples were obtained in 8 patients at both collection times: time 1, at the start of treatment, and time 2, after one month, at the end of treatment.

Figure 3 shows the composition of the gut microbiota, classified by phylum, for each of the samples. No differences were found in alpha diversity (Shannon index), nor in beta diversity (unweigthed and weighted Unifrac). Next, a paired relative abundance analysis was carried out. In this case, significant differences (p<0.05) were found in two ASVs, corresponding to bacteria belonging to the phylum Proteobacteria. The FDR corrected values were found to be non-significant.

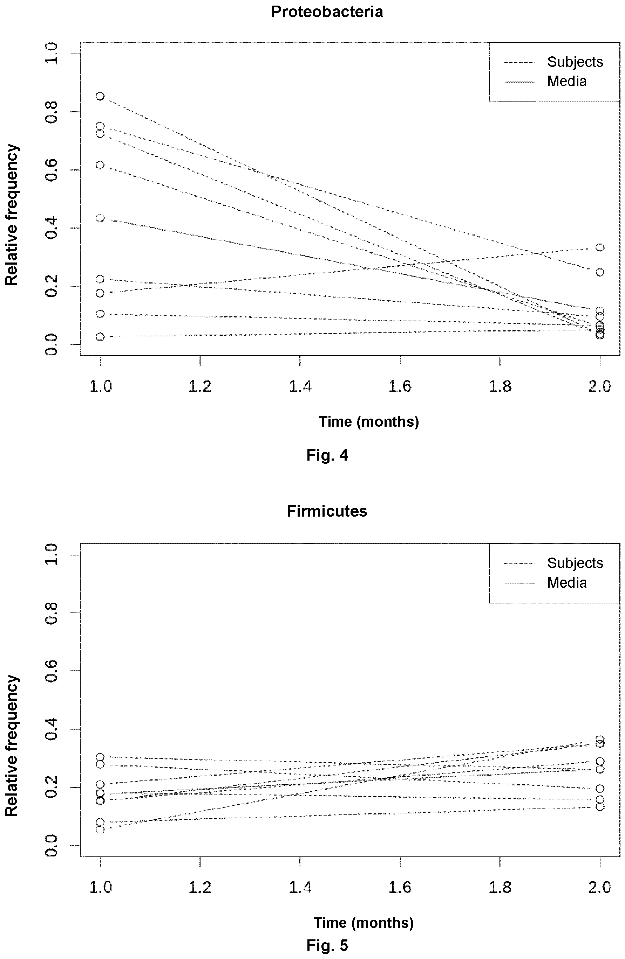

Figure 4 shows the relative frequency for the phylum Proteobacteria, at time 1 and time 2, for each of the subjects, where a significant trend of decreasing relative proportions of Proteobacteria can be observed at time 2. Similarly, although no significant differences were obtained, the relative proportions for each of the subjects at time 1 and time 2 for the phyla Firmicutes and Bacteroidetes are shown.

### Adverse effects

The most frequent symptom (44.4%) was occasional mild colicky abdominal pain. This coincided with the first interventions where faeces were of less consistency. No patient requested to leave the study and it was well tolerated. One patient (11.1%) experienced discomfort and nausea with the instillation manoeuvres, with no other repercussions. No severe adverse effects were reported.

### Conclusions

The majority of patients treated produced functional faeces as described, with progressive improvement of results over time. The majority of faeces were considered normal compared to those before the ileostomy was performed. The rate of patients who did not achieve optimal results is related to the expected population frequency of severe LARS.

Initially, the lower amount of instilled composition prevented adequate defecation frequency, but this improved with increasing volume applied. Subsequently, as the instilled volume was increased, diarrhea symptoms increased, leading to a worsening of the LARS assessment. Finally, in the last week of intervention, after adjusting and stabilizing the dosage for each patient, the best results in terms of LARS were obtained.

All treated patients produced biologically active faeces at the end of the study. After continued use of the composition of the invention, changes in the faecal microbiota were observed after the intervention. Initially, it was observed that the population of the phylum Proteobacteria was very high. This is compatible with the dysbiosis and inflammatory state of the colon (exclusion colitis) [23]. In the faecal sample analyzed after completion of the intervention, a significant decrease in the same Proteobacteria phylum was observed. This finding, together with the improvement in aqueous absorptive capacity (observed in the normalization of the Bristol scale at the same instillation volume), supports the capacity of the composition of the invention to rehabilitate the intestinal microbiota, improve the colonic inflammatory state, and therefore treat exclusion colitis.

The ability of the composition of the invention to form functional faeces, to restore physiological peristaltic and defecatory function, to rehabilitate the intestinal microbiota and to treat exclusion colitis by restoring nutrition of the colorectal mucosa is thus demonstrated.

### References

1. Healthcare Quality Improvement Partnership (2015) National Bowel Cancer Audit Report 2018.
2. Beamish EL, Johnson J, Shaw EJ, Scott NA, Bhowmick A, Rigby RJ. Loop ileostomy-mediated fecal stream diversion is associated with microbial dysbiosis. Gut Microbes 2017; 8: 467-78.
3. Rombey, T., Panagiotopoulou, I.G., Hind, D. and Fearnhead, N.S. (2019), Preoperative bowel stimulation prior to ileostomy closure to restore bowel function more quickly and improve postoperative outcomes: a systematic review. Colorectal Dis, 21: 994-1003.
4. Harries RL, Ansell J, Codd RJ, Williams GL. A systematic review of Clostridium difficile infection following reversal of ileostomy. Colorectal Dis 2017; 19: 881-7.
5. Kabir SI, Kabir SA, Richards R, et al. Pathophysiology, clinical presentation and management of diversion colitis: a review of current literature. International Journal of Surgery 2014; 12: 1088-92.
6. Roe AM, Warren BF, Brodribb AJ, et al. Diversion colitis and involution of the defunctioned anorectum. Gut 1993; 34: 382-5.
7. Levy E, Palmer DL, Frileux P, Parc R, Huguet C, Loygue J. Inhibition of upper gastrointestinal secretions by reinfusion of succus entericus into the distal small bowel. A clinical study of 30 patients with peritonitis and temporary enterostomy. Ann Surg. 1983; 198(5):596-600.
8. Maeda K, HashimotoM, Koh J, Yamamoto O, Hosoda Y, Morikawa Y. The use of an ileostomy connector to diminish the frequency of defecation prior to ileostomy closure in patients with a pelvic pouch. Surg Today. 1995;25(7):657-661.
9. P. Sharma, R. Davidson, J. Davidson, C. Keane, C. Liu, S. R. Ritchie et al. Novel chyme reinfusion device for gastrointestinal fistulas and stomas: feasibility study. Br J Surg. 2020. DOI:10.1002/bjs.11516.
10. Luceri C, et al. Effect of butyrate enemas on gene expression profiles and endoscopic / histopathological scores of diverted colorectal mucosa: A randomized trial. Dig Liver Dis (2015) 2016; 48(1): 27-33.
11. Caltabiano C, Maximo FR, Spadari AP, da Conceicao Miranda DD, Serra MM, Ribeiro ML, Martinez CA. 5-aminosalicylic acid (5-ASA) can reduce levels of oxidative DNA damage in cells of colonic mucosa with and without fecal stream. Dig Dis Sci 2011; 56: 1037-1046.
12. Lim AG, Langmead FL, Feakins RM, Rampton DS. Diversion colitis: a trigger for ulcerative colitis in the in-stream colon? Gut 1999; 44: 279-282.
13. Kentaro Tominaga, Kenya Kamimura, Kazuya Takahashi, et al. Diversion colitis and pouchitis: A mini-review. World J Gastroenterol 2018; 24(16): 1734-1747.
14. De Oliveira-Neto JP, de Aguilar-Nascimento JE. Intraluminal irrigation with fibers improves mucosal inflammation and atrophy in diversion colitis. Nutrition 2004; 20: 197-199.
15. Martelucci J. Low anterior resection syndrome: a treatment algorithm. Dis Colon Rectum 2016; 59:79-81.
16. Heaton KW, Radvan J, Cripps H, et al. Defecation frequency and timing, and faeces form in the general population: a prospective study. Gut 1992; 33: 818-824.
17. Lewis SJ, Heaton KW. Faeces Form Scale as a Useful Guide to Intestinal Transit Time. Scandinavian Journal of Gastroenterology. 1997; 32(9): 920-924.
18. Qin, J., et al., 2010. A human gut microbial gene catalogue established by metagenomic sequencing. Nature 464 (7285), 59-65.
19. Ishiguro, E., Haskey, N., & Campbell, K. (2018). The Gut Microbiota. Gut Microbiota, 17-39. doi:10.1016/b978-0-12-810541-2.00002-6.
   Trejo-Márquez, M.A., Lira-Vargas, A.A., & Pascual-Bustamante, S. (2016). Fibre for the future: properties and benefits. In M.E. Ramirez Ortiz (Ed.). Functional Foods Today. Barcelona, Spain: OmniaScience.1-34.
21. Oken M, Creech R, Tormey D, et al. Toxicity and response criteria of the Eastern Cooperative Oncology Group. Am J Clin Oncol. 1982; 5: 649-655.
22. Amin MB, Edge S, Greene F, et al. (Eds.). AJCC Cancer Staging Manual (8th edition). Springer International Publishing: American Joint Commission on Cancer; 2017.
23. Shin NR, Whon TW, Bae JW. Proteobacteria: microbial signature of dysbiosis in gut microbiota. Trends Biotechnol. 2015; 33: 496-503.
24. Middelbos IS, Fastinger ND, Fahey GC Jr. Evaluation of fermentable oligosaccharides in diets fed to dogs in comparison to fiber standards. J Anim Sci. 2007 Nov;85(11):3033-44. doi: 10.2527/jas.2007-0080.
25. Boelens PG, Heesakkers FF, Luyer MD, van Barneveld KW, de Hingh IH, Nieuwenhuijzen GA, Roos AN, Rutten HJ. Reduction of postoperative ileus by early enteral nutrition in patients undergoing major rectal surgery: prospective, randomized, controlled trial. Ann Surg. 2014 Apr;259(4):649-55. doi: 10.1097/SLA.0000000000000288.
26. EP 3632222 A1 Beijing Ruiqianjing Science And Tech Development Co Ltd 08/04/2020
27. Abrisqueta J. et al. Efferent loop stimulation prior to ileostomy closure. Cirugia Española, 13/11/2012, Vol. 91, N° 1, Pages 50-52.

## Claims

1. A composition for its use in a method of treatment for the restoration of functional faeces formation and physiological defecatory function in patients with temporary diverting ileostomy comprising at least one prebiotic oligosaccharide and a soluble cellulose derivative.

2. Composition according to claim 1 wherein the prebiotic oligosaccharide is inulin and/or galacto-oligosaccharide.

3. Composition according to the preceding claim wherein the inulin is oligofructose and/or polyfructose.

4. Composition according to any of the preceding claims wherein the soluble cellulose derivative is methyl cellulose, hydroxyl propyl methyl cellulose and/or carboxy methyl cellulose.

5. Composition according to any of the preceding claims, further comprising at least one humectant.

6. Composition according to the preceding claim wherein the humectant is polydextrose, mucilage, gum, starch, alginate and/or agar.

7. Composition according to any of the preceding claims comprising a cellulose derivative (50-70%), inulin (30-50%), GOS (0-5%) and humectant (0-10%).

8. Composition according to any of the preceding claims further comprising insoluble fibre.

9. Composition according to the preceding claim wherein the insoluble fibre is cellulose, insoluble hemicellulose and/or lignin.

10. Composition according to any of the preceding claims further comprising Medium Chain Triglycerides and/or antioxidant.

11. Composition according to any of the preceding claims further comprising electrolytes, calcium phosphate and/or ferric phosphate.

12. Composition according to any of the preceding claims, further comprising contrast media for MRI and/or X-ray scanning.

13. Use of the composition according to any of claims 1 to 12 for the manufacture of a medicament.

14. Use of the composition according to claim 13 wherein the medicament is intended for the restoration of functional faeces formation and physiological defecatory function.

15. A method of obtaining data useful for predicting or prognosticating Severe Anterior Resection Syndrome in patients with temporary bypass ileostomy who have previously been administered the composition of claims 1 to 12, comprising:
a) assessing defecatory function according to the LARS score, and
b) classifying defecatory function as No LARS; minor LARS or major LARS.

16. The method for predicting or prognosticating Severe Anterior Resection Syndrome in patients with temporary bypass ileostomy who have previously been administered the composition of claims 1 to 12, comprising steps (a) - (b) of claim 15 and further comprising:
c) assigning the individual with a faeces score of more than 30 points on the LARS score, LARS major, to the group of patients at high risk for Severe Anterior Resection Syndrome and who are not ideal candidates for bowel reconstruction.
